# EUROPÄISCHE PATENTSCHRIFT

(11) **EP 0 493 417 B1**
(45) Veröffentlichungstag und Bekanntmachung des Hinweises auf die Patenterteilung: **02.03.1994**
(21) Anmeldenummer: 90913390.2
(22) Anmeldetag: 24.08.1990
(51) Int. Cl.: G01N 21/00, G01N 21/75

(54) **VERFAHREN ZUR BESTIMMUNG SEHR GERINGER MENGEN AN HERBIZIDEN AUF DER BASIS VON TRIAZINVERBINDUNGEN**
PROCESS FOR DETERMINING VERY SMALL QUANTITIES OF HERBICIDES BASED ON TRIAZINE COMPOUNDS
PROCEDE DE DETERMINATION DE TRES FAIBLES QUANTITES D'HERBICIDES BASES SUR DES COMPOSES TRIAZINE

(30) Priorität: 20.09.1989 DE 3931360
(43) Veröffentlichungstag der Anmeldung: 08.07.1992
(73) Patentinhaber: HOELZLE & CHELIUS GMBH, D-63234 Neu-Isenburg (DE)
(72) Erfinder: AFFONSO, Alvaro, D-6380 Bad Homburg v.d.H. (DE)
(74) Vertreter: Lauer, Dieter, Dr.
(86) Internationale Anmeldenummer: EP9001414
(87) Internationale Veröffentlichungsnummer: WO9104481

(56) Entgegenhaltungen:
- US-A- 3 535 044
- US-A- 3 751 167
- US-A- 4 304 996
- US-A- 4 775 794
- DERWENT'S ABSTRACT, Nr. 87-77 691/11, SU 1 242 778, publ. woche 8711

## Beschreibung

Die vorliegende Erfindung betrifft ein Verfahren zur Bestimmung sehr geringer Mengen an Herbiziden auf der Basis von Triazinverbindungen in flüssigen oder festen Substanzen oder deren Gemischen auf photometrischem Wege.

Die Bestimmung von Herbiziden, Insektiziden und dgl. stieß seit einigen Jahrzehnten auf zunehmendes Interesse der Fachwelt. Indessen wurde die Verunreinigung von Trinkwasser mit Pestiziden in den letzten Jahren zu einem akuten Problem. Die Tatsache, daß Hunderte von Pestiziden nicht nur in Europa und in USA, sondern auch in der übrigen Welt frei verkäuflich sind, ließ die Bestimmung des Ausmaßes der Verunreinigung nicht nur des Erdbodens, sondern auch des Trinkwassers durch diese Substanzen besonders dringlich erscheinen.

So versuchen die Trinkwasserverordnung vom Mai 1986 und die nachfolgenden Verordnungen des Bundesgesundheitsministeriums Grenzen für die Anwesenheit von Pestiziden im Trinkwasser festzulegen, und zwar soll deren Menge 0,0005 mg/l als Gesamtsumme der Herbizide, Pestizide und deren Metabolite im Trinkwasser nicht übersteigen. Die Bestimmung derart geringer Mengen erfordert jedoch die Anwendung von Analysenverfahren und -geräten, die nicht bei allen Untersuchungsstellen durchführbar und verfügbar sind. Eine grobe Übersicht über die Anwesenheit von Pestizidverunreinigungen im Trinkwasser in der Bundesrepublik hat gezeigt, daß die am häufigsten vorkommenden und gefährlichsten Verunreinigungen die Triazin-Herbizide Atrazin und Simazin sind. Aus diesem Grunde wurde bereits versucht, einfache und schnelle Methoden zur Bestimmung des Gesamtgehalts der Triazinverbindungen in Wasser-, Boden- oder Schlammproben zu finden.

So ist aus der europäischen Patentanmeldung 0 242 225 ein Verfahren zur Bestimmung von Verunreinigungen, u.a. von Herbiziden auf der Basis von Triazinverbindungen, in wäßrigen Flüssigkeiten bekannt. Der zu untersuchenden Flüssigkeit wird ein Elektronentransfervermittler zugegeben und die Mischung in eine Meßzelle überführt, die ein Mittel mit lebenden Bakterien enthält, deren Aktivität angeregt und an der Elektrode in der Zelle gemessen wird.

Ein anderes Bestimmungsverfahren für Triazin-Herbizide ist in dem Buch "Wasseranalyse" v. W. Fresenius, R.E Quentin, W. Schneider, Springer Verlag, Berlin, Heidelberg, New York 1988, Seite 589 beschrieben. Die Extraktion der zu untersuchenden Substanz erfolgt mit Dichlormethan, Adsorption an Aktivkohle/Silikagel-Kolonne, Elution mit Dichlormethan/ Toluol/Aceton-Gemisch, Verdampfung des Lösungsmittels, Auflösung des Rückstandes in Toluol und anschließende Gaschromatographie mit einem molekularspezifischen Detektor.

Beide genannten Verfahren erfordern indessen die Verwendung spezieller Vorrichtungen und spezieller Substanzen, verbunden mit entsprechenden Vorsichtsmaßnahmen im Falle der Verwendung von Bakterien. Darüber hinaus ist die genannte Extraktion und die Herstellung der für die Untersuchung geeigneten Probe sehr aufwendig.

Aus Chemical Abstracts 51, 656 (1957), Journal of Agricultural and Food Chemistry, 14, 70 (1966) und Journal of Agricultural and Food Chemistry 16, 284 (1968) sind colorimetrische Verfahren zur Bestimmung von Triazinherbiziden bekannt, wobei wäßrige Lösungen dieser Herbizide der Pyridin-Alkali-Methode unterworfen und die das Reaktionsprodukt enthaltende klare Mischung untersucht wurde. Eine Nacharbeitung des in den vorgenannten Literaturstellen beschriebenen Verfahrens ergab jedoch folgende Nachteile:
1. Das bekannte Nachweisverfahren ist bei der von den zuständigen Behörden festgelegten maximalen Grenze von 0,0005 mg/l nicht empfindlich. Gerade beim Arbeiten mit größeren Mengen an Wasserproben reichte die Empfindlichkeit dieses Nachweisverfahrens nicht aus.
2. Die Autoren empfehlen die colorimetrische Messung der unbeständigen gelben Farbe des Absorptionsmaximums des Reaktionsprodukts im sichtbaren Wellenlängenbereich von 435 - 440 nm. Infolge des kontinuierlichen Verblassens dieser Farbe liegt keine Stabilität derselben vor und eine Reproduzierbarkeit des bekannten Nachweisverfahrens ist nicht gegeben. Deshalb wurde bereits versucht, durch weitere Umsetzung des Reaktionsproduktes mit Cyanoacetat oder Barbitursäure eine andere, beständigere Farbbildung zu erreichen. Die Empfindlichkeit der Nachweisreaktion wurde nur geringfügig verbessert.
3. Die angeführten Literaturstellen geben keine handliche Methode zur Herstellung der Untersuchungsproben von Wasser, Böden, Sedimenten oder Schlämmen an.

Aufgabe der vorliegenden Erfindung war es daher, ein Verfahren zur Bestimmung sehr geringer Mengen an Herbiziden auf der Basis von Triazinverbindungen, vorzugsweise von 2-Chlor-4-ethylamino-6-isopropylamino-1,3,5-triazin (common name: Atrazin), 2,4-Bis(ethylamino)-6-chlor-1,3,5-triazin (common name: Simazin) und 2-Chlor-4,6-bis(isopropylamino)-1,3,5-triazin (common name: Propazin) zu finden, das noch bei einer sehr geringen Konzentration von 0,0001 mg/l Triazin-Herbizid-Verunreinigung eine genaue und empfindliche qualitative und quantitative Bestimmung gewährleistet, einfach und leicht durchführbar sowie reproduzierbar ist. Weiterhin sollte es nur die Verwendung üblicher Nachweisinstrumente wie Spektralphotometer erfordern, mit dem die Untersuchungslaboratorien in der Regel ausgerüstet sind. Darüber hinaus sollte die zur Herstellung der zu analysierenden Probe erforderliche Extraktion handlich und derart durchführbar sein, daß der Extrakt direkt für die Bestimmung des Triazin-Herbizid-Gehalts verwendet werden kann.

Erfindungsgemäß wurde festgestellt, daß diesen Zielen und Aufgaben ein Verfahren zur Bestimmung sehr geringer Mengen an Herbiziden auf der Basis von Triazinverbindungen in flüssigen oder festen Substanzen oder deren Gemischen auf photometrischem Wege gerecht wird, bei dem das Herbizid mit einem wasserunlöslichen oder wasserschwerlöslichen organischen Lösemittel extrahiert, der Extrakt mit einer 5 bis 80 vol.-%igen, vorzugsweise 60 bis 70 vol.-%igen wäßrigen Lösung von Pyridin oder deren Derivaten versetzt, die erhaltene Lösung auf eine Temperatur von 50 bis 105°C, vorzugsweise 80 bis 100°C während 10 bis 50 min, vorzugsweise 20 bis 40 min erhitzt, nach dem Abkühlen eine konzentrierte wäßrige alkalische Lösung in einer der Pyridinlösung gleichen Menge zugegeben und das in der abgetrennten Pyridinschicht enthaltene Reaktionsprodukt der Triazinverbindung mittels UV-Spektroskopie im Wellenlängenbereich von 400 bis 200 nm gemessen wird.

Das erfindungsgemäße Verfahren erlaubt eine qualitative und quantitative Bestimmung von herbiziden Triazinverbindungen in Mengen von 0,0005 mg/l und weniger. Dies wird durch die erfindungsgemäße Kombination einer Extraktion der in der flüssigen oder festen Substanz oder in dem Gemisch aus fester und flüssiger Substanz enthaltenen Triazinverbindung mit einem geeigneten organischen Lösemittel, das wasserunlöslich oder wasserschwerlöslich ist, mit der erfindungsgemäßen Abwandlung der Reaktion einer Triazinverbindung mit Pyridin in Gegenwart von Alkali derart, daß nicht nur ein weiteres, farbloses, in der Pyridinschicht stabiles Reaktionsprodukt mit einer starken Absorption im UV-Spektralbereich von 400 bis 200 nm gebildet wird, sondern auch eine glatte Trennung der dieses Reaktionsprodukt enthaltendenden Pyridinschicht von der konzentrierten wäßrigen alkalischen Schicht erfolgt, erzielt.

Als Beispiele für flüssige Substanzen, deren Verunreinigung an Triazin-Herbiziden nach dem erfindungsgemäßen Verfahren bestimmt werden können, seien außer Trinkwasser noch Grundwasser, Oberflächenwasser, Brauchwasser und dgl. genannt.

Als wasserunlösliches oder wasserschwerlösliches organisches Lösemittel wird erfindungsgemäß vorteilhaft ein wasserunlöslicher oder wasserschwerlöslicher aliphatischer oder ein aromatischer Ester der Essigsäure, vorzugsweise Amylacetat, verwendet. Als Beispiel für einen geeigneten aromatischen Ester der Essigsäure sei Benzylacetat genannt.

Zur Bestimmung der Triazin-Herbizide 2-Chlor-4-ethylamino-6-isopropylamino-1,3,5-triazin (common name: Atrazin), 2,4-Bis(ethylamino)-6-chlor-1,3,5-triazin (common name: Simazin) und 2-Chlor-4,6-bis (isopropylamino)-1,3,5-triazin (common name: Propazin) wird erfindungsgemäß vorzugsweise das bei 355 nm liegende UV-Absorptionsmaximum verwendet.

Gemäß einer vorzugsweisen Ausführungsform des erfindungsgemäßen Verfahrens beträgt das Verhältnis des Volumens der die Triazinverbindung enthaltenden flüssigen Substanz zum Volumen des zur Extraktion verwendeten organischen, wasserunlöslichen oder wasserschwerlöslichen Lösemittels 100 bis 500 : 1, vorzugsweise 200 bis 250 : 1, wobei die Extraktion der flüssigen Substanz mit dem Lösemittel unter Zuhilfenahme eines schnellaufenden oder Hochgeschwindigkeitsrührers, vorzugsweise bei einer Rührgeschwindigkeit von 20.000 U/min, durchgeführt wird. Hierdurch wird die zu extrahierende Flüssigkeit mit dem organischen, wasserunlöslichen oder wasserschwerlöslichen Lösemittel besonders innig durchmischt und die Phasengrenzfläche vergrößert, so daß der Extraktionsvorgang schneller ablaufen kann. Der weitere Vorteil der genannten Ausführungsform liegt darin, daß nur geringe Mengen an Lösemittel zur Extraktion erforderlich sind, was die Durchführung des erfindungsgemäßen Bestimmungsverfahrens vereinfacht und zugleich zum Umweltschutz beiträgt.

Gemäß einer weiteren Ausführungsform des erfindungsgemäßen Verfahrens wird die die herbizide Triazinverbindung enthaltende feste Substanz, beispielsweise Bodenproben, Sedimente oder das die herbizide Triazinverbindung enthaltende Gemisch aus flüssiger und fester Substanz wie wäßrige Schlämme vor der Extraktion mit dem wasserunlöslichen oder wasserschwerlöslichen organischen Lösemittel in an sich bekannter Weise (siehe Deutsche Einheitsverfahren zur Wasser-, Abwasser- und Schlammuntersuchung, Bd. III, Verlag Chemie, 1989) mit einer Säure aufgeschlossen und anschließend mit destilliertem Wasser stark verdünnt.

Als konzentrierte wäßrige alkalische Lösung wird in dem erfindungsgemäßen Verfahren eine konzentrierte wäßrige Alkalilauge oder Erdalkalilauge, eine konzentrierte wäßrige Alkalicarbonatlösung oder Erdalkalicarbonatlösung oder eine konzentrierte wäßrige organische Base, beispielsweise Benzyldimethylammoniumhydroxid, verwendet. Vorzugsweise wird jedoch als konzentrierte wäßrige alkalische Lösung eine 2 N bis 10 N Kaliumhydroxidlösung, vorteilhaft eine 9 N Kaliumhydroxidlösung verwendet.

Gemäß einer vorteilhaften Ausführungsform des erfindungsgemäßen Verfahrens wird das in Wasser enthaltene Herbizid auf der Basis von Triazinverbindungen statt der Extraktion mit dem wasserunlöslichen oder wasserschwerlöslichen organischen Lösemittel einer Festphasenextraktion unterworfen. Hierbei wird das Herbizid zunächst an einem geeigneten Sorbens adsorbiert und anschließend mit der wäßrigen Lösung von Pyridin oder dessen Derivaten eluiert, die erhaltene Lösung erhitzt, nach dem Abkühlen die konzentrierte wäßrige alkalische Lösung in einer der Pyridinlösung gleichen Menge zugegeben und das in der abgetrennten Pyridinschicht enthaltene Reaktionsprodukt der Triazinverbindung mittels UV-Spektroskopie im Wellenlängenbereich von 400 bis 200 mm gemessen. Das Festphasenextraktionverfahren weist den Vorteil auf, daß kein organisches Lösemittel zur Extraktion der Herbizide auf der Basis von Triazinverbindungen aus der zu untersuchenden Probe erforderlich ist.

Bei der Durchführung des Verfahrens gemäß der vorhin beschriebenen Ausführungsform wird zur vorteilhaften kontinuierlichen Bestimmung der Summe der im Trinkwasser enthaltenen Herbizide auf der Basis von Triazinverbindungen eine dreiteilige Vorrichtung verwendet, die aus einem Extraktionsteil, einem Elutionsteil und einem analytischen Teil besteht.

500 ml- bis 1000 ml-Proben des zu untersuchenden Trinkwassers werden aufeinanderfolgend durch an sich bekannte, mit einem festen Sorbens gefüllte Patronen oder Säulen, sogenannte Festphasenextraktionspatronen oder -säulen, die an einer automatisch rotierenden Platte oder Karussel angeordnet sind, geleitet. Durch das feste Sorbens, das aus einem chemisch modifizierten Kieselgel besteht, werden die betreffenden Herbizide aus dem Trinkwasser extrahiert, so daß eine sogenannte Festphasenextraktion erfolgt. Anschließend wird in dem Elutionsteil der Vorrichtung aus jeder Patrone oder Säule das Herbizid direkt mit 1 bis 2 ml der vorzugsweise 70 vol%-igen wäßrigen Pyridinlösung eluiert. Das Eluat bzw. die herbizidhaltige Pyridinlösung wird in einem kapillaren Spiral-Glasrohr innerhalb eines thermostatisch kontrollierten Metallblocks innerhalb eines Temperaturbereiches von 50 - 105 °C für ungefähr 5 Minuten erhitzt. Anschließend werden 1 bis 2 ml der konzentrierten wäßrigen alkalischen Lösung zur abgekühlten Pyridinschicht gegeben und in der Mikrokuvette eines UV-Spektrophotometers, welches den analytischen Teil der Vorrichtung darstellt, die Konzentration des Herbizids spektroskopisch wie angegeben bestimmt.

### Beispiele:

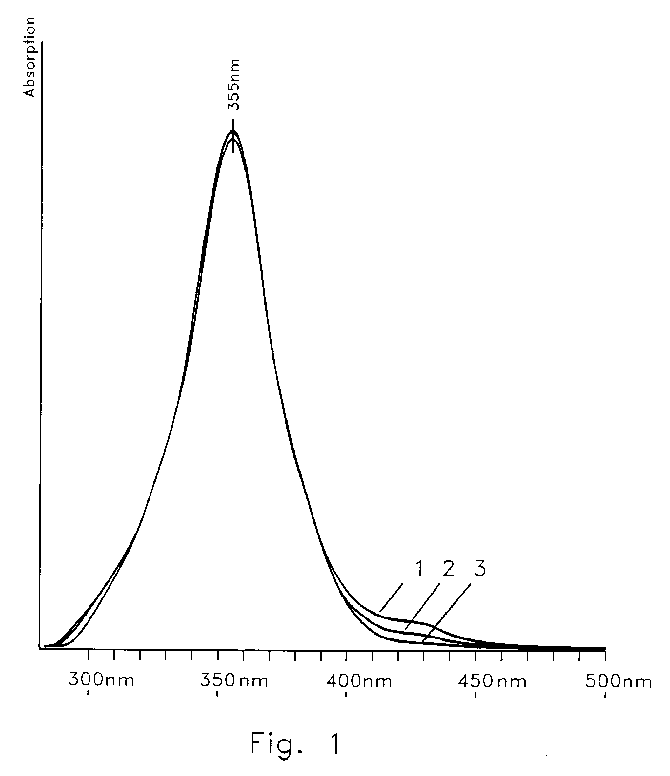

A. Eine Lösung von Atrazin in Amylacetat in einer Konzentration von 0,0005 mg/l wurde zu einem bestimmten Volumen einer 70 vol.-%igen wäßrigen Pyridinlösung gegeben und 30 Minuten lang auf eine Temperatur von 100°C erhitzt. Nach dem Abkühlen wurde die Mischung mit einer 9N KOH-Lösung in einer Volummenge versetzt, die derjenigen von Pyridin entsprach. Die Pyridinschicht ließ sich nach kräftigem Schütteln leicht abtrennen.
   Die Pyridinschicht wurde abgetrennt und nach dem Zusatz von KOH die UV-Spektralanalyse vorgenommen. Das Spektrum zeigte einen sehr kleinen Peak bei 435 nm und einen sehr starken Peak bei 355 nm. Der Peak bei 435 nm verschwand innerhalb von 3 Minuten, während der Peak bei 355 nm noch nach 20 Minuten stabil blieb, siehe Figur 1. Somit ist das erfindungsgemäße Verfahren zur Bestimmung geringer Mengen an Herbiziden auf der Basis von Triazinverbindungen sehr empfindlich und reproduzierbar. Der Grund hierfür dürfte in der Bildung einer in der abgetrennten Pyridinschicht stabilen, im UV-Bereich bei 355 nm absorbierenden Verbindung bestehen. Die von führenden Autoren für die colorimetrische Bestimmung von Triazinderivaten benutzte gelb gefärbte Verbindung mit schwacher Absorption ist für Messungen mit üblichen Spektralphotometern ungeeignet. Der weitere Vorteil des erfindungsgemäßen Bestimmungsverfahrens liegt darin, daß die zu untersuchende Probe mit einem organischen Lösemittel extrahiert wird, das keine oder nur eine geringe Wasserlöslichkeit aufweist. Hierdurch können Wasser, Schlämme oder Sedimente direkt extrahiert werden. Die Empfindlichkeit des erfindungsgemäßen Bestimmungsverfahrens erlaubt den Nachweis noch von 0,0001 mg/l des Triazinderivats, insbesondere Atrazin, was in der Größenordnung des gaschromatographischen Nachweises liegt.
   Die Figur 1 zeigt das Absorptionsspektrum der gemäß dem erfindungsgemäßen Verfahren erhaltenen Pyridinschicht nach verschiedenen Zeitabständen. Absorptionspeaks bei 355 nm
   1 nach 5 min
   2 nach 10 min
   3 nach 20 min

   Hieraus ist die besonders hohe Stabilität des für die Bestimmung der genannten herbiziden Triazinderivate verwendeten Peaks bei 355 nm ersichtlich.

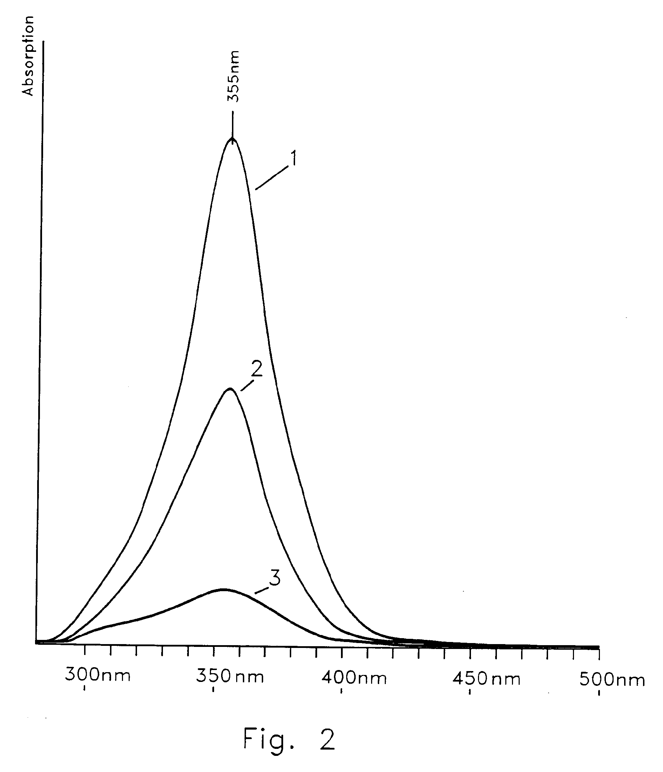

   Die Figur 2 zeigt das Absorptionsspektrum der gemäß dem erfindungsgemäßen Verfahren erhaltenen Pyridinschicht mit unterschiedlichen Konzentrationen an Atrazin bei 355 nm.
   1 1,0 µg = 0,001 mg
   2 0,5 µg = 0,0005 mg
   3 0,1 µg = 0,0001 mg
   1 µg = 10⁻⁶g
   Hieraus geht besonders die Linearität und die extreme Empfindlichkeit der Absorptionswerte hervor.
B. Herstellung einer Eichkurve für reines Atrazin: Es wurden Standard-Atrazin-Lösungen in Amylacetat hergestellt. Der Konzentrationsbereich wurde von 0,0001 mg/l bis 0,0005 mg/l gewählt. In allen Fällen betrug das Volumen des Amylacetats als Lösemittel 0,1 ml. Diese Lösungen wurden in Küvetten mit Teflonstopfen und Schraubverschluß, die 1 ml 70% vol.-%ige wäßrige Pyridinlösung enthielten, gegeben. Sie wurden in einem Thermoblock mit elektronischem Regulator, der beispielsweise bis zu 17 Küvetten aufnehmen kann, bei einer Temperatur von 100°C 20 bis 40 Minuten lang erhitzt. Nach dem Abkühlen werden die Küvetten mit 1 ml 9N KOH versetzt und geschüttelt. Es bildete sich sofort eine gut abtrennbare Pyridinschicht.
   Etwa 1,0 ml dieser Schicht wurden abpipettiert und in die Quarzküvette eines Spektralphotometers gegeben, das Absorptionsmaximum bei 355 nm gemessen und die Eichkurve aufgenommen.
   In der gleichen Weise können auch die Eichkurven der anderen Triazinderivate sowie von Mischungen derselben hergestellt werden.
C. Bestimmung der herbiziden Triazinderivate in Trinkwasser.
   5 l Trinkwasser wurden 5 min lang mit 20 ml Amylacetat extrahiert. Hierbei wurde ein Hochgeschwindigkeitsrührer mit einer Umdrehungszahl von 20.000 U/min verwendet. Das Amylacetat wird hierdurch in sehr kleine Tröpfchen durch die ganze Wasserprobe hindurch verteilt. Nach 30 minütigem Stehen wird der Amylacetatextrakt, der zur Oberfläche schwimmt, abgetrennt. 6 ml dieses Extraktes werden in ein Teströhrchen mit Vakuumanschluß abpipettiert. Diese Teströhrchen werden nach Anschluß an eine Vakuumpumpe (ca. 10 mm Vakuum) im Thermoblock erhitzt, bis alles Amylacetat entfernt ist. Der Rückstand wurde mit 0,2 ml Amylacetat aufgenommen, 0,1 ml abpipettiert und dieses abpipettierte Volumen wie unter B beschrieben weiterverarbeitet. Die Absorption der Pyridinschicht im UV-Wellenlängenbereich wurde gemessen und die Menge des Triazinderivats durch Vergleich mit einer Eichkurve bestimmt.

## Patentansprüche

1. Verfahren zur Bestimmung sehr geringer Mengen an Herbiziden auf der Basis von Triazinverbindungen in flüssigen oder festen Substanzen oder deren Gemischen auf photometrischem Wege, dadurch gekennzeichnet, daß das Herbizid mit einem wasserunlöslichen oder wasserschwerlöslichen organischen Lösemittel extrahiert, der Extrakt mit einer 5 bis 80 vol.-%igen, vorzugsweise 60 bis 70 vol.-%igen wäßrigen Lösung von Pyridin oder dessen Derivaten versetzt, die erhaltene Lösung auf eine Temperatur von 50 bis 105°C, vorzugsweise 80 bis 100°C während 10 bis 50 min, vorzugsweise 20 bis 40 min erhitzt, nach dem Abkühlen eine konzentrierte wäßrige alkalische Lösung in einer der Pyridinlösung gleichen Menge zugegeben und das in der abgetrennten Pyridinschicht enthaltene Reaktionsprodukt der Triazinverbindung mittels UV-Spektroskopie im Wellenlängenbereich von 400 bis 200 nm gemessen wird.

2. Verfahren nach Anspruch 1, dadurch gekennzeichnet, daß das bei 355 nm liegende Absorptionsmaximum für die Bestimmung der Triazinverbindungen 2-Chlor-4-ethylamino-6-isopropylamino-1,3,5-triazin, 2,4-Bis(ethylamino)-6-chlor-1,3,5-triazin und 2-Chlor-4,6-bis(isopropylamino)-1,3,5-triazin verwendet wird.

3. Verfahren nach Anspruch 1 oder 2, dadurch gekennzeichnet, daß als wasserunlösliches oder wasserschwerlösliches organisches Lösemittel ein wasserunlöslicher oder wasserschwerlöslicher alipha-tischer oder aromatischer Ester der Essigsäure, vorzugsweise Amylacetat verwendet wird.

4. Verfahren nach einem oder mehreren der Ansprüche 1 bis 3, dadurch gekennzeichnet, daß das Verhältnis des Volumens der die Triazinverbindung enthaltenden flüssigen Substanz zum Volumen des zur Extraktion verwendeten organischen wasserunlöslichen oder wasserschwerlöslichen Lösemittels 100 bis 500 : 1, vorzugsweise 200 bis 250 : 1 beträgt und die Extraktion unter Zuhilfenahme eines schnellaufenden Rührers oder Hochgeschwindigkeitsrührers, vorzugsweise bei einer Rührgeschwindigkeit von 20 000 U/min, durchgeführt wird.

5. Verfahren nach einem oder mehreren der Ansprüche 1 bis 3, dadurch gekennzeichnet, daß die die Triazinverbindung enthaltende Festsubstanz oder das Gemisch aus fester und flüssiger Substanz mit einer Säure aufgeschlossen, mit destilliertem Wasser stark verdünnt und die erhaltene Suspension mit dem wasserunlöslichen oder wasserschwerlöslichen organischen Lösemittel extrahiert wird.

6. Verfahren nach einem oder mehreren der Ansprüche 1 bis 5, dadurch gekennzeichnet, daß als konzentrierte wäßrige alkalische Lösung eine konzentrierte wäßrige Alkalilauge oder Erdalkalilauge, eine konzentrierte wäßrige Alkalicarbonatlösung oder Erdalkalicarbonatlösung oder eine konzentrierte wäßrige organische Base verwendet wird.

7. Verfahren nach Anspruch 6, dadurch gekennzeichnet, daß als organische Base Benzyldimethylammoniumhydroxid verwendet wird.

8. Verfahren nach Anspruch 6, dadurch gekennzeichnet, daß als konzentrierte wäßrige alkalische Lösung eine 2 N bis 10 N Kaliumhydroxidlösung, vorzugsweise eine 9 N Kaliumhydroxidlösung verwendet wird.

9. Verfahren nach einem oder mehreren der Ansprüche 1 bis 3 und 6 bis 8, dadurch gekennzeichnet, daß das in Wasser enthaltene Herbizid statt der Extraktion mit einem organischen Lösemittel einer Festphasenextraktion unterworfen wird, indem das Herbizid zunächst an einem Sorbens adsorbiert und anschließend mit der wäßrigen Lösung von Pyridin oder dessen Derivaten eluiert, die erhaltene Lösung erhitzt, nach dem Abkühlen die konzentrierte wäßrige alkalische Lösung in einer der Pyridinlösung gleichen Menge zugegeben und das in der abgetrennten Pyridinschicht enthaltene Reaktionsprodukt der Triazinverbindung mittels UV-Spektroskopie im Wellenlängenbereich von 400 bis 200 nm gemessen wird.

10. Verfahren nach Anspruch 9, dadurch gekennzeichnet, daß für die kontinuierliche Bestimmung des im Trinkwasser enthaltenen Herbizids 500 ml- bis 1000 ml- Proben durch in einer karusselartigen Vorrichtung angeordnete und bewegte, mit 100 bis 500 mg eines festen Sorbens gefüllte Patronen geleitet, das adsorbierte Herbizid direkt aus jeder Patrone mit 1 bis 2 ml der 5 bis 80 vol.-%igen, vorzugsweise 60 bis 70 vol.-%igen wäßrigen Pyridinlösung eluiert, die erhaltene Lösung auf eine Temperatur von 50 bis 105 °C, vorzugsweise 80 bis 100 °C während 3 bis 5 min, vorzugsweise 4 min in einem kapillaren Spiral-Glasrohr erhitzt, nach einer Abkühlzeit von 1 bis 2 min die konzentrierte wäßrige alkalische Lösung zugegeben und das in der abgetrennten Pyridinschicht enthaltende Reaktionsprodukt spektroskopisch gemessen wird.

## Claims

1. Method for determining very small amounts of herbicides based on triazine compounds in liquid or solid materials or mixtures thereof by a photometric technique, characterized in that the herbicide is extracted with a water-insoluble or sparingly water-soluble organic solvent, the extract is admixed with a from 5 to 80% by volume strength, preferably from 60 to 70% by volume strength, aqueous solution of pyridine or derivatives thereof, the solution obtained is heated to a temperature of from 50 to 105°C, preferably from 80 to 100°C, for from 10 to 50 min, preferably from 20 to 40 min, after cooling a concentrated aqueous alkaline solution is added in an amount equal to that of the pyridine solution, the pyridine layer is separated off, and the reaction product of the triazine compound contained therein is measured by means of UV spectroscopy in the wavelength range from 400 to 200 nm.

2. Method according to Claim 1, characterized in that the absorption maximum at 355 nm is used for the determination of the triazine compounds 2-chloro-4-ethylamino-6-isopropylamino-1,3,5-triazine, 2,4-bis(ethylamino)-6-chloro-1,3,5-triazine and 2-chloro-4,6-bis(isopropylamino)-1,3,5-triazine.

3. Method according to Claim 1 or 2, characterized in that the water-insoluble or sparingly water-soluble organic solvent used is a water-insoluble or sparingly water-soluble aliphatic or aromatic ester of acetic acid, preferably amyl acetate.

4. Method according to one or more of Claims 1 to 3, characterized in that the volume ratio of the liquid containing the triazine compound to the water-insoluble or sparingly water-soluble organic solvent used for the extraction is from 100 : 1 to 500 : 1, preferably from 200 : 1 to 250 : 1, and the extraction is carried out with the aid of a fast-running stirrer or high-speed stirrer, preferably at a stirring speed of 20,000 rpm.

5. Method according to one or more of Claims 1 to 3, characterized in that the solid containing the triazine compound, or the mixture of solid and liquid are digested with an acid and greatly diluted with distilled water and the suspension obtained is extracted with the water-insoluble or sparingly water-soluble organic solvent.

6. Method according to one or more of Claims 1 to 5, characterized in that the concentrated aqueous alkaline solution used is a concentrated aqueous alkali metal hydroxide solution or alkaline earth metal hydroxide solution, a concentrated aqueous alkali metal carbonate solution or alkaline earth metal carbonate solution or a concentrated aqueous organic base.

7. Method according to Claim 6, characterized in that the organic base used is benzyldimethylammonium hydroxide.

8. Method according to Claim 6, characterized in that the concentrated aqueous alkaline solution used is a from 2 N to 10 N potassium hydroxide solution, preferably a 9 N potassium hydroxide solution.

9. Method according to one or more of Claims 1 to 3 and 6 to 8, characterized in that the herbicide contained in water is, instead of the extraction with an organic solvent, subjected to a solid phase extraction, in which the herbicide is first adsorbed on a sorbent and subsequently eluted with the aqueous solution of pyridine or derivatives thereof, the solution obtained is heated, after cooling the concentrated aqueous alkaline solution is added in an amount equal to that of the pyridine solution, the pyridine layer is separated off, and the reaction product of the triazine compound contained therein is measured by means of UV spectroscopy in the wavelength range from 400 to 200 nm.

10. Method according to Claim 9, characterized in that, for the continuous determination of herbicide contained in drinking water, 500 ml to 1,000 ml samples are fed through cartridges packed with from 100 to 500 mg of a solid sorbent and arranged and moving in a carousel-type device, the adsorbed herbicide is directly eluted from each cartridge with from 1 to 2 ml of a from 5 to 80 % by volume strength, preferably from 60 to 70% by volume strength aqueous pyridine solution, the solution obtained is heated to a temperature of from 50 to 105°C, preferably from 80 to 100°C, for from 3 to 5 min, preferably 4 min, in a capillary glass spiral, after a cooling time of from 1 to 2 min the concentrated aqueous alkaline solution is added, the pyridine layer is separated off, and the reaction product contained therein is measured spectroscopically.

## Revendications

1. Procédé de détermination, par voie photométrique, de très faibles quantités d'herbicides à base de triazines dans des substances liquides ou solides ou dans leurs mélanges, caractérisé en ce qu'on soumet l'herbicide à une extraction à l'aide d'un solvant organique insoluble dans l'eau ou peu soluble dans l'eau, on ajoute à l'extrait une solution aqueuse contenant 5 à 80 % en volume, avantageusement 60 à 70 % en volume de pyridine ou de ses dérivés, on chauffe la solution ainsi obtenue à une température de 50 à 105°C, avantageusement 80 à 100°C pendant 10 à 50 minutes, avantageusement 20 à 40 minutes, on ajoute après le refroidissement, en une quantité égale à celle de la solution de pyridine, une solution alcaline aqueuse concentrée et l'on mesure par spectroscopie dans l'ultra-violet, dans le domaine des longeurs d'ondes de 400 à 200 nm, le produit de réaction de la triazine contenu dans la couche de pyridine séparée.

2. Procédé selon la revendication 1, caractérisé en ce qu'on utilise le maximum d'absorption se situant à 355 nm pour la détermination des triazines que sont la 2-chloro-4-éthylamino-6-isopropylamino-1,3,5-triazine, la 2,4-bis (éthylamino)-6-chloro-1,3,5-triazine et la 2-chloro-4,6-bis (isopropylamino)-1,3,5-triazine.

3. Procédé selon la revendication 1 ou 2, caractérisé en ce qu'on utilise comme solvant organique insoluble dans l'eau ou peu soluble dans l'eau un ester aliphatique ou aromatique, insoluble dans l'eau ou peu soluble dans l'eau, de l'acide acétique, avantageusement l'acétate d'amyle.

4. Procédé selon l'une ou plusieurs des revendications 1 à 3, caractérisé en ce que le rapport du volume de la substance liquide contenant la triazine au volume du solvant organique, insoluble dans l'eau ou peu soluble dans l'eau, utilisé pour l'extraction vaut de 100 à 500:1, avantageusement 200 à 250:1, et en ce qu'on effectue l'extraction à l'aide d'un agitateur rapide ou d'un agitateur tournant à grande vitesse, avantageusement à une vitesse d'agitation de 20 000 tours par minute.

5. Procédé selon une ou plusieurs des revendications 1 à 3, caractérisé en ce que la substance solide conteant la triazine ou le mélange de substances solides et substances liquides est soumis à une attaque effectuée à l'aide d'un acide, à une forte dillution par de l'eau distillée et en ce que la suspension ainsi obtenue est extraite à l'aide du solvant organique insoluble dans l'eau ou peu soluble dans l'eau.

6. Procédé selon une ou plusieurs des revendications 1 à 5, caractérisé en ce qu'on utilise comme solution alcaline aqueuse une lessive alcaline ou alcalino-terreuse aqueuse concentrée, une solution aqueuse concentrée de carbonate alcalin ou de carbonate alcalino-terreux ou une base organique aqueuse concentrée.

7. Procédé selon la revendication 6, caractérisé en ce qu'on utilise comme base organique l'hydroxyde de benzyldiméthylammonium.

8. Procédé selon la revendication 6, caractérisé en ce qu'on utilise comme solution alcaline aqueuse concentrée une solution 2 N à 10 N d'hydroxyde de potassium, avantageusement une solution 9 N d'hydroxyde de potassium.

9. Procédé selon une ou plusieurs des revendications 1 à 3 et 6 à 8, caractérisé en ce qu'au lieu d'une extraction à l'aide d'un solvant organique, on soumet l'herbicide contenu dans de l'eau à une extraction en phases solides en adsorbant tout d'abord l'herbicide sur un sorbant puis en éluant avec la solution aqueuse de pyridine ou de ses dérivés, en chauffant la solution obtenue et, après son refroidissement, en lui ajoutant, en une quantité égale à celle de la solution de pyridine, la solution alcaline aqueuse concentrée et en mesurant, par spectroscopie dans l'ultra-violet dans le domaine des longueurs d'ondes de 400 à 200 nm, le produit de réaction de la triazine contenu dans la couche de pyridine séparée.

10. Procédé selon la revendication 9, caractérisé en ce que, pour la détermination en continu de l'herbicide contenu dans de l'eau potable on envoie des échantillons de 500 ml à 1 000 ml traverser des cartouches emplies de 100 à 500 mg d'un sorbant solide, agitées et disposées dans un dispositif du genre carrousel, en ce qu'on élue directement de chaque cartouche l'herbicide adsorbé, en utilisant 1 à 2 ml de la solution aqueuse de pyridine à 5 à 80 % en volume, avantageusement à 60 à 70 % en volume, en ce qu'on chauffe la solution ainsi obtenue à une température de 50 à 105°C, avantageusement 80 à 100°C durant 3 à 5 minutes, avantageusement durant 4 minutes dans un tube capillaire en verre en spirale et, après un temps de refroidissement de 1 à 2 minutes, on ajoute la solution alcaline aqueuse concentrée et l'on effectue une mesure spectroscopique du produit de réaction contenu dans la couche de pyridine séparée.
